# EUROPEAN PATENT APPLICATION

(11) **EP 1 431 894 A1**
(43) Date of publication of application: **23.06.2004**
(21) Application number: 02800283.0
(22) Date of filing: 30.09.2002
(51) Int. Cl.: G06F 17/60

(54) **MEDICAL INFORMATION DISTRIBUTION METHOD**

(30) Priority: 28.09.2001 JP 2001304121
(71) Applicant: Olympus Corporation, Tokyo 151-0072 (JP)
(72) Inventor: KONDO, Seiji, Hachioji-shi, Tokyo 192-0045 (JP)
(74) Representative: von Hellfeld, Axel, Dr. Dipl.-Phys.
(86) International application number: PCT/JP2002/010173
(87) International publication number: WO 2003/030047

(57) **Abstract**

There is disclosed a constitution comprising a step of transmitting medical information obtained by use of apparatuses and/or consumables such as reagent associated consumables to obtain the medical information from a patient or a specimen to an information management institution from a medical care/test institution, a step of searching a database by the use of the medical information received from the medical care/test institution to return a search result to the medical care/test institution, and changing the medical information received from the medical care/test institution into a predetermined format to store the information in the database in the information management institution, a step of transmitting an inquiry for the clinical data to the information management institution from a research institution, and a step of searching the database based on the inquiry received from the research institution to return the search result to the research institution from the information management institution, and the apparatuses and/or the consumables such as the reagent associated consumables to obtain the medical information are supplied to the medical care/test institution from the information management institution at a charge or free of charge.

## Description

### Technical Field

The present invention relates to a distribution method of medical information.

### Background Art

In recent years, in genome drug production by use of a gene information technique which has flourished, it has been very important to acquire clinical data, especially a relation between a disease and a gene arrangement, or a relation between a function of a drug and the gene arrangement for development. When the patient's gene data is quickly obtained in a large amount, the development of a competitive drum is largely influenced.

Moreover, it has been recognized that the relations between the genes and the diseases or the drugs have been gradually clarified even in hospitals or inspection centers and that when the relations are quickly taken into medical treatments, reductions of medical costs or labor costs result.

However, there is not any valuable distribution route in a method of acquiring the patient's gene data, and each pharmaceutical institution individually cooperates with the hospital or the inspection center to acquire the data. There is a problem that it is difficult to acquire high-quality data in large amounts by individual handling.

Moreover, in the present situation, gene inspection requires large amounts of inspection and labor costs, further there is not any method of checking a relation with medical information required for making use of the data, and therefore the inspection has not spread. Since the inspection and labor costs increase, the gene information is supplied to the pharmaceutical institution in a small amount, and this has formed a vicious circle to inhibits the spreading.

### Disclosure of Invention

An object of the present invention is to provide a distribution method of medical information, which is capable of lowering prices of inspection apparatuses and consumables such as reagent associated consumables to largely lower a cost of inspection.

Moreover, another object of the present invention is to provide a distribution method of medical information, in which a research institution can easily acquire a large amount of patient data constantly updated to up-to-the-minute information and in which a medical care/test institution can take in latest medicine or disease data from the research institution to obtain latest analysis results.

According to a first aspect of the present invention, there is provided a distribution method of medical information, in which a single or a plurality of medical care/test institutions, an information management institution, and a single or a plurality of research institutions distribute the medical information with one another, the distribution method of the medical information, comprising:
a step of transmitting the medical information obtained by use of apparatuses and/or consumables such as reagent associated consumables to obtain the medical information from a patient or a specimen to the information management institution from the medical care/test institution;
a step of searching a database by the use of the medical information received from the medical care/test institution to return a search result to the medical care/test institution, and changing the medical information received from the medical care/test institution into a predetermined format to store clinical data in the database in the information management institution;
a step of transmitting an inquiry for the clinical data to the information management institution from the research institution; and
a step of searching the database based on the inquiry received from the research institution to return the search result to the research institution from the information management institution,
wherein the apparatuses and/or the consumables such as the reagent associated consumables to obtain the medical information are supplied to the medical care/test institution from the information management institution at a charge or free of charge.

Moreover, according to a second aspect of the present invention, there is provided a distribution method of medical information, in which a single or a plurality of medical care/test institutions, an information management institution, a single or a plurality of research institutions, and a single or a plurality of manufacturing/selling institutions of apparatuses and/or consumables such as a reagent associated consumable distribute the medical information with one another, the distribution method of the medical information, comprising:
a step of transmitting the medical information obtained by use of apparatuses and/or consumables such as reagent associated consumables to obtain the medical information from a patient or a specimen to the information management institution from the medical care/test institution;
a step of searching a database by the use of the medical information received from the medical care/test institution to return a search result to the medical care/test institution, and changing the medical information received from the medical care/test institution into a predetermined format to store clinical data in the database in the information management institution;
a step of transmitting an inquiry for the clinical data to the information management institution from the research institution; and
a step of searching the database based on the inquiry received from the research institution to return the search result to the research institution from the information management institution,
wherein the apparatuses and/or the consumables such as the reagent associated consumables to obtain the medical information are supplied to the medical care/test institution from the information management institution at a charge or free of charge, and a rebate is supplied to the manufacturing/selling institutions of the apparatuses and/or the consumables such as the reagent associated consumables from the information management institution.

Furthermore, according to a third aspect of the present invention, there is provided a distribution method of medical information, in which a single or a plurality of medical care/test institutions, an information management institution, and a single or a plurality of research institutions distribute the medical information with one another, the distribution method of the medical information, comprising:
a step of sending a specimen to the information management institution from the medical care/test institution;
a step of obtaining the medical information with respect to the sent specimen in the information management institution;
a step of searching a database by use of the medical information to return a search result to the medical care/test institution, and changing the obtained medical information into a predetermined format to store clinical data in the database;
a step of transmitting an inquiry for the clinical data to the information management institution from the research institution; and
a step of searching the database based on the inquiry received from the research institution to return the search result to the research institution from the information management institution.

Additionally, according to a fourth aspect of the present invention, the distribution method of the medical information relates to the distribution method of the medical information according to the first aspect of the present invention, and further comprises:
a step of transmitting specific medicine data to the information management institution from the research institution; and
a step of updating the database by the received medicine data in the information management institution.

Moreover, according to a fifth aspect of the present invention, the distribution method of the medical information relates to the distribution method of the medical information according to the second aspect of the present invention, and further comprises:
a step of transmitting specific medicine data to the information management institution from the research institution; and
a step of updating the database by the received medicine data in the information management institution.

Furthermore, according to a sixth aspect of the present invention, the distribution method of the medical information relates to the distribution method of the medical information according to the third aspect of the present invention, and further comprises:
a step of transmitting specific medicine data to the information management institution from the research institution; and
a step of updating the database by the received medicine data in the information management institution.

Moreover, the distribution method of the medical information according to a seventh aspect of the present invention relates to the distribution method of the medical information according to the first aspect of the present invention, and a fixed global IP address is attached to the apparatus to obtain the medical information.

Furthermore, the distribution method of the medical information according to an eighth aspect of the present invention relates to the distribution method of the medical information according to the second aspect of the present invention, and a fixed global IP address is attached to the apparatus to obtain the medical information.

Additionally, the distribution method of the medical information according to a ninth aspect of the present invention relates to the distribution method of the medical information according to the third aspect of the present invention, and a fixed global IP address is attached to the apparatus to obtain the medical information.

Moreover, the distribution method of the medical information according to a tenth aspect of the present invention relates to the distribution method of the medical information according to the first aspect of the present invention, and further comprises a step of subjecting the medical information to an interpolation preventive measure and encryption.

Furthermore, the distribution method of the medical information according to an eleventh aspect of the present invention relates to the distribution method of the medical information according to the second aspect of the present invention, and further comprises a step of subjecting the medical information to an interpolation preventive measure and encryption.

Additionally, the distribution method of the medical information according to a twelfth aspect of the present invention relates to the distribution method of the medical information according to the third aspect of the present invention, and further comprises a step of subjecting the medical information to an interpolation preventive measure and encryption.

It is to be noted that the "apparatus" mentioned in the present embodiment refers to means for use to obtain various biological inspection data which is medical information required for researching a medicine, new biological material and the like which can be objects of the research, and refers to the apparatus at least a part of which is mechanized and preferably automated. The "reagent associated consumables" mentioned in the present embodiment broadly refer to reagents which are consumables for use in the biological inspection, and can be optional reagents in response to a required biological reaction. The consumables sometimes refer to containers for reaction which are integrally used together with the reagents and discarded or collected as the consumables after one or more uses. These "apparatus" and "reagent" are sold or lent alone or both and are objects of purchase and sale, and therefore the "apparatus" and/or the "reagent" is distributed even in the present invention. It is to be noted that the biological inspection in the present invention can be an optional inspection in accordance with purposes such as diagnosis of the medical information and medical effects (e.g., healing capacity, preventive capacity value, etc.), and can be, for example, an optional combination of a genetic (allergy, infection, transplantation immunity, etc.) inspection, a biochemical (enzyme, hormone, etc.) inspection and the like.

### Brief Description of Drawings

FIG. 1 is a diagram schematically showing a flow of information among three of an medical care/test institution and an information management institution and a drug creation research institution in a first embodiment of the present invention;
FIG. 2 is a diagram showing a flow at a time when information, apparatus and/or reagent, and consideration are mutually exchanged among a plurality of medical care/test institutions, a plurality of drug creation research institutions, and the information management institution;
FIG. 3 is a diagram showing a constitution in which an apparatus and/or reagent manufacturing/selling institution is disposed in addition to the three including medical care/test institution, information management institution, and drug creation research institution; and
FIG. 4 is a diagram schematically showing a flow of information in a case which a specimen sent from the medical care/test institution is measured in the information management institution in a second embodiment of the present invention.

### Best Mode for Carrying Out the Invention

An embodiment of the present invention will be described hereinafter in detail with reference to the drawings. In a distribution method of medical information according to the present invention, it is first assumed that information, apparatus and/or reagent, and consideration are exchanged among three including a medical care/test institution, information management institution, and drug creation research institution. Here, the medical care/test institution means, for example, a hospital, inspection center and the like, the information management institution means an information search company, information analysis service company and the like, and the drug creation research institution means, for example, a pharmaceutical maker, public research institute and the like.

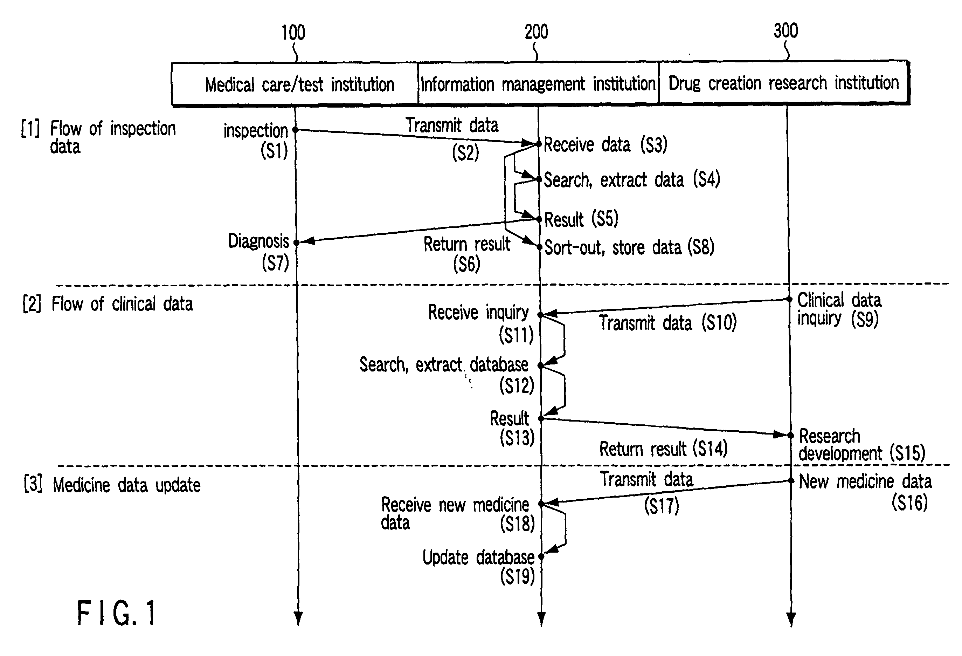

FIG. 1 is a diagram schematically showing a flow of information among three including the medical care/test institution, information management institution, and drug creation research institution. In the present embodiment, all flows of some pieces of classified information are made with respect to one database or a plurality of databases which mutually closely cooperate.

In FIG. 1, [1] Flow of inspection data will be described hereinafter. First, a medical care/test institution 100 performs inspection, for example, by use of the apparatus and/or the reagent supplied from an information management institution 200 (S1), and transmits medical data (medical information) obtained by the inspection to the information management institution 200 (S2). The information management institution 200 receives the data (S3), performs a database search, extraction process (S4), and acquires a certain result (S5). This result is returned as auxiliary information effective for diagnosis to the medical care/test institution 100 (S6). The medical care/test institution 100 performs the diagnosis by the use of the received result (S7).

Moreover, the information management institution 200 individually sorts out the data received from the medical care/test institution 100, changes the data into a predetermined format, and stores the data (S8). This can be used in protecting privacy of inspection data.

Next, [2] Flow of clinical data will be described. First, when an inquiry concerning the clinical data is generated (S9), a drug creation research institution 300 transmits the inquiry to the information management institution 200 (S10). The information management institution 200 receives the inquiry (S11), searches and extracts the database (S12), and acquires some result (S13). The information management institution 200 returns the acquired result to the drug creation research institution 300 (S14). The drug creation research institution 300 performs research/development by the use of the received result (S15). It is to be noted that the privacy of the returned result needs to be sufficiently protected.

Next, [3] Update of medicine data will be described. First, on acquiring new medicine data (S16), the drug creation research institution 300 transmits the data to the drug creation research institution 300 (S17). The information management institution 200 receives the new medicine data (S18), and updates the database (S19). At this time, the reception of the medicine data includes charged and published reception, and includes reception free of charge, or reception by contract with an institution which receives the clinical data by the flow of [2]. The information management institution may also act so as to acquire the medicine data by itself.

It is to be noted that an order in which the above-described [1], [2], [3] can occur is not limited to the above-described order and that, for example, [3] Update of medicine data, [2] Flow of clinical data, and [1] Flow of inspection data may also occur in order.

A relation among the medical care/test institution, information management institution, and drug creation research institution, and a function of the information management institution, required for realizing the distribution method of the medical information according to the present embodiment will be described hereinafter.

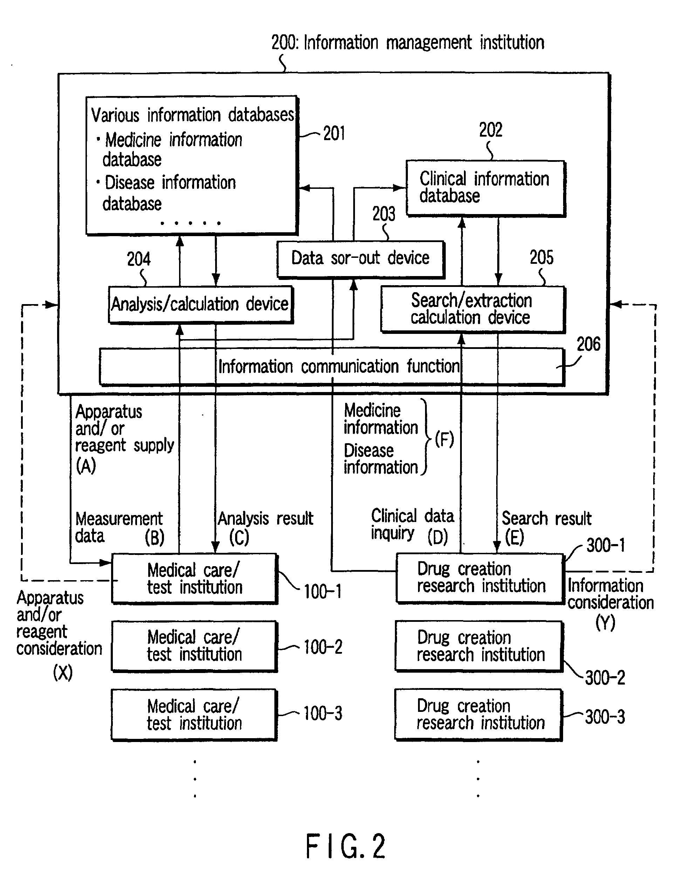

FIG. 2 shows a flow at a time when a plurality of medical care/test institutions 100-1, 2, 3, ... (hereinafter described only with respect to 100-1 which is an example), a plurality of drug creation research institutions 300-1, 2, 3, ... (hereinafter described only with respect to 300-1 which is an example), and the information management institution 200 mutually exchange information, apparatus, and consideration. Moreover, as shown in FIG. 2, the information management institution 200 includes various information databases 201 including a medicine information database or a disease information database, a clinical information database 202, a data sort-out device 203, an analysis/calculation device 204, a search/extraction calculation device 205, and an information communication function 206.

When the information management institution 200 includes the above-described elements, the following functions can be realized.
(a) The information management institution 200 supplies the apparatus and/or reagent to obtain the medical information, and attached consumables, for example, with respect to the medical care/test institution 100-1 ((A) of FIG. 1). This is performed regardless of charges. In a charged case, the information management institution 200 receives an apparatus consideration (X).
(b) The information management institution 200 receives measurement data ((B) of FIG. 1) obtained by performing the inspection by the use of the apparatus and/or the reagent by the medical care/test institution 100-1 from the medical care/test institution 100-1 via networks such as the Internet. The analysis/calculation device 204 analyzes the data by the use of the various information databases 201 in accordance with purposes, and analysis results ((C) of FIG. 1) are returned to the medical care/test institution 100-1. The consideration is not required concerning the information supply or has a low amount inversely proportional to an information amount and/or quality.
(c) The information management institution 200 sorts out the data received via the network in (b) by the use of attached information (e.g., race, gender, age, clinical history, etc.) by the data sort-out device 203 to add or update the data with respect to the clinical information database 202.
(d) The information management institution 200 searches the clinical information database 202 in a range permitted by contract and the like by the search/extraction calculation device 205 with respect to the inquiry for the clinical data ((D) of FIG. 1), for example, from the drug creation research institution 300-1, and returns a search result (E) to the drug creation research institution 300-1. An access to the database is permitted in a range determined by the contract. An information consideration (Y) is received with respect to the supply of the information.
(e) Furthermore, the information management institution 200 adds medicine information, disease information (F) and the like sent from the drug creation research institution 300-1 contracted in (d) and other pharmaceutical institutions to the corresponding database by the data sort-out device 203, so that latest information can be constantly supplied. These information are supplied regardless of charges, and the information management institution 200 is sometimes a main body to acquire the information.

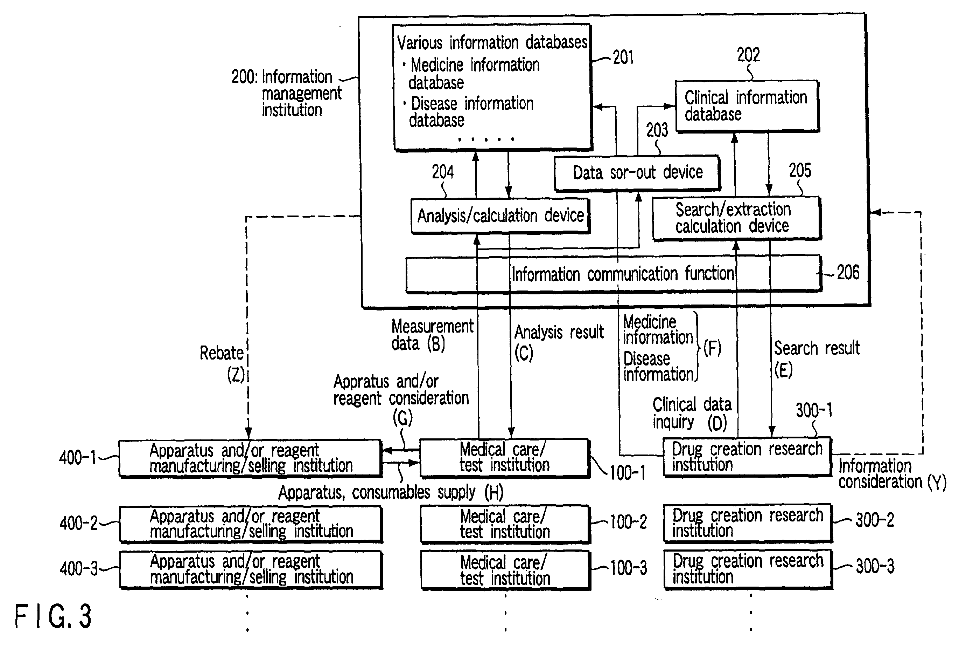

Next, among the above-described functions (a) to (e), for (a), in addition to three institutions including the medical care/test institution, information management institution, and drug creation research institution, the apparatus and/or reagent manufacturing/selling institution is supposed to be disposed. This embodiment will be described hereinafter with reference to FIG. 3. As shown in FIG. 3, a plurality of apparatus and/or reagent manufacturing/selling institutions 400-1, 2, 3, ... (hereinafter described only with respect to 400-1 which is the example) are added to the constitution of FIG. 2.

The apparatus and/or reagent manufacturing/selling institution 400-1 supplies the apparatus and/or the reagent from which the medical information is obtained and the attached consumables to the medical care/test institution 100-1 ((H) of FIG. 2). This is performed regardless of the charges. In the charged case, the apparatus and/or reagent manufacturing/ selling institution 400-1 receives an apparatus and/or reagent consideration (G) from the medical care/test institution 100-1.

The medical care/test institution 100-1 performs measurement by the use of the apparatus supplied from the apparatus and/or reagent manufacturing/selling institution 400-1, and sends the obtained measurement data (B) to the information management institution 200. On the other hand, the information management institution 200 supplies a rebate (Z) to the apparatus and/or reagent manufacturing/selling institution 400-1 by the contract.

As described above, when the medical care/test institution 100, information management institution 200, drug creation research institution 300, and apparatus and/or reagent manufacturing/selling institution 400-1 if necessary exchange the information, apparatus and/or reagent, and consideration, the following advantages are obtained.
1. Costs can largely be lowered with respect to the apparatus and/or the reagent and the consumables and the analysis in the medical care/test institution.
2. A large amount of clinical data can easily be obtained in the drug creation research institution.

It is to be noted that there is not any restriction as to the apparatus and/or the reagent for use in the medical care/test institution to obtain the medical information, but physical changes in light absorption, fluorescence, electric conductivity, temperature, viscosity and the like or the specimen or a carrier which has reacted to the specimen are measured by the apparatus and/or the reagent. More concretely, this includes an apparatus which measures an immune reaction of the specimen, an apparatus which measures genome/gene information of the specimen and/or reagent associated consumables for the measurement. These apparatuses are connected to a device connected to the network integrally or by cable or radio. Alternatively, the device is separately prepared. Furthermore, a fixed IP address defined by the existing IPv4 or IPv6 supposed to be a mainstream from now on is attached. When IPv4 or IPv6 is handled, and all the apparatuses can individually be handled, reliability of transmitted data from the information management institution is enhanced. It is also necessary to incorporate encryption or interpolation preventive measure for preventing interpolation or wiretapping of the data. Here, a case in which the rebate is not carried out with respect to the apparatus and/or the reagent is also considered. In this case, the medical care/test institution cannot lower the cost with respect to the analysis, but the drug creation research institution has the advantage. There is an advantage that a latest analysis result by a latest database can constantly be received at a low price.

Moreover, database processing devices installed in the information management institution (the above-described data sort-out device 203, analysis/calculation device 204, search/extraction calculation device 205) may be any types of devices as long as the data is processed. Preferable examples include a large-sized computer, work station, personal computer and the like. The database processing device may not be owned by the information management institution, and may also be rental or leased. Furthermore, an installation place is not limited to the inside of a building possessed by the information management institution. For safety of the database, at least a part of the information or a duplicate of an information storage member may also be stored separately from the database processing device.

A content of the database held/managed in the information management institution changes with the inspection which is an object.

### (Second Embodiment)

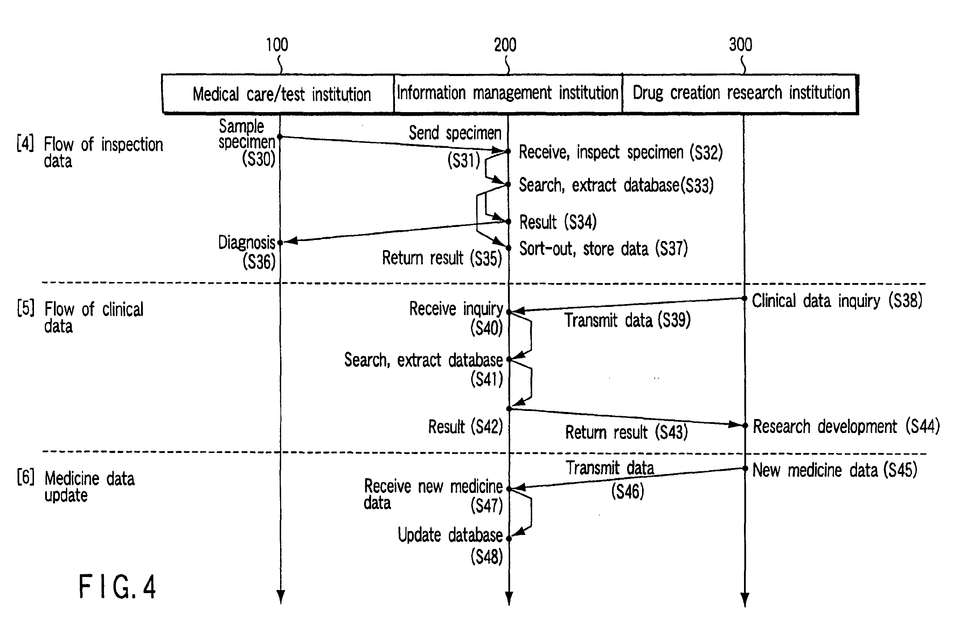

A second embodiment of the present invention will be described hereinafter. FIG. 4 is a diagram schematically showing a flow of information in a case in which a specimen sent from the medical care/test institution is measured in the information management institution.

In FIG. 4, [4] Flow of inspection data will be described hereinafter. First, the medical care/test institution 100 samples a specimen (S30), and sends the sampled specimen to the information management institution 200 (S31). The specimen may be genome electron data sufficient for the inspection of the specimen. The information management institution 200 receives and inspects the specimen (S32), searches and extracts the database by the use of the result at this time (S33), and acquires a certain result (S34). This result is returned as auxiliary information effective for diagnosis to the medical care/test institution 100 via the network, telegraph, or document (S35). The medical care/test institution 100 makes use of the received result and performs the diagnosis (S36).

Moreover, the information management institution 200 individually sorts out the data acquired by the database search, changes the data into the predetermined format, and stores the data (S37). This can be useful for the privacy protection of the inspection data.

Moreover, details of [5] Flow of clinical data (S38 to S44) and [6] Update of medicine data (S45 to S48) are as described with reference to FIG. 1.

It is to be noted that the order in which the above-described [4], [5], [6] can occur is not limited to the above-described order and that, for example, [6] Update of medicine data, [5] Flow of clinical data, and [4] Flow of inspection data may also occur in this order.

It is to be noted that the present invention is not limited to the above-described various examples, and can be further variously modified based on an essential concept of the present invention. For example, for all the information for use in the distribution in the present invention, one type or a plurality of types of networks among the Internet, LAN, satellite circuit, telephone circuit and the like are used. Moreover, for the information to be distributed, with reference to optional information to be concealed, including private information related to the privacy of the patient or the specimen, and attribute information usable for special purposes, such as race, religion, and family history, the information for the distribution is appropriately removed, canceled, masked, encrypted, and dummied or otherwise. Accordingly, the information may also be processed into specified information specified partially or to such an extent that required data is contained, and distributed. In the distribution method of the medical information, institutions in optional locations can participate regardless of domestic/international inspections in a case where international regulations such as appropriate import/export provisions are observed. Therefore, nationalities of the medical care/test institution, information management institution, and drug creation research institution can be an optional combination (e.g., a combination of the medical care/test institution of a developing country, the research institution of an advanced country, and the information management institution in Japan). The same type of institutions or related institutions can be a combination of institutions having a plurality of different nationalities (e.g., a combination of the respective medical care/test institutions of an origin country of an infection and infection import/export countries) as long as the institution s satisfy the same purpose. Based on the scope of the present invention, in addition to the above-described various types of institutions, third institutions such as a support institution, entrusted institution, and branch station may also be interposed. As the case may be, a supplier of the medical information may be the patient or a deputy such as patient's family. Moreover, based on the scope of the present invention, the present invention can be regarded as software for controlling each means to execute miscellaneous functions on the network for use in the above-described distribution method of the medical information, program, or a medium (magnetic optical disk, computerized program) in which the software or the program is stored or coded).

### Industrial Applicability

According to the present invention, an information management institution, or an apparatus and/or reagent manufacturing/selling institution if necessary is capable of entirely obtaining a profit, and therefore the profit does not have to be gained by sales of an apparatus. Accordingly, prices of inspection apparatuses and consumables can be reduced, and a cost of inspection can largely be lowered.

Moreover, a research institution can easily acquire a large amount of patient data constantly updated to latest information. Moreover, when latest medicine or disease data is taken in from the research institution, a medical care/test institution can constantly obtain a latest analysis result to use the result in diagnosis or medical treatment.

## Claims

1. A distribution method of medical information, in which a single or a plurality of medical care/test institutions, an information management institution, and a single or a plurality of research institutions distribute the medical information with one another, the distribution method of the medical information, comprising:
a step of transmitting the medical information obtained by use of apparatuses and/or consumables such as reagent associated consumables to obtain the medical information from a patient or a specimen to the information management institution from the medical care/test institution;
a step of searching a database by the use of the medical information received from the medical care/test institution to return a search result to the medical care/test institution, and changing the medical information received from the medical care/test institution into a predetermined format to store clinical data in the database in the information management institution;
a step of transmitting an inquiry for the clinical data to the information management institution from the research institution; and
a step of searching the database based on the inquiry received from the research institution to return the search result to the research institution from the information management institution,
wherein the apparatuses and/or the consumables such as the reagent associated consumables to obtain the medical information are supplied to the medical care/test institution from the information management institution at a charge or free of charge.

2. A distribution method of medical information, in which a single or a plurality of medical care/test institutions, an information management institution, a single or a plurality of research institutions, and a single or a plurality of manufacturing/selling institutions of apparatuses and/or consumables such as a reagent associated consumable distribute the medical information with one another, the distribution method of the medical information, comprising:
a step of transmitting the medical information obtained by use of apparatuses and/or consumables such as reagent associated consumables to obtain the medical information from a patient or a specimen to the information management institution from the medical care/test institution;
a step of searching a database by the use of the medical information received from the medical care/test institution to return a search result to the medical care/test institution, and changing the medical information received from the medical care/test institution into a predetermined format to store clinical data in the database in the information management institution;
a step of transmitting an inquiry for the clinical data to the information management institution from the research institution; and
a step of searching the database based on the inquiry received from the research institution to return the search result to the research institution from the information management institution,
wherein the apparatuses and/or the consumables such as the reagent associated consumables to obtain the medical information are supplied to the medical care/test institution from the information management institution at a charge or free of charge, and a rebate is supplied to the manufacturing/selling institutions of the apparatuses and/or the consumables such as the reagent associated consumables from the information management institution.

3. A distribution method of medical information, in which a single or a plurality of medical care/test institutions, an information management institution, and a single or a plurality of research institutions distribute the medical information with one another, the distribution method of the medical information, comprising:
a step of sending a specimen to the information management institution from the medical care/test institution;
a step of obtaining the medical information with respect to the sent specimen in the information management institution;
a step of searching a database by use of the medical information to return a search result to the medical care/test institution, and changing the obtained medical information into a predetermined format to store clinical data in the database;
a step of transmitting an inquiry for the clinical data to the information management institution from the research institution; and
a step of searching the database based on the inquiry received from the research institution to return the search result to the research institution from the information management institution.

4. The distribution method of the medical information according to claim 1, further comprising:
a step of transmitting specific medicine data to the information management institution from the research institution; and
a step of updating the database by the received medicine data in the information management institution.

5. The distribution method of the medical information according to claim 2, further comprising:
a step of transmitting specific medicine data to the information management institution from the research institution; and
a step of updating the database by the received medicine data in the information management institution.

6. The distribution method of the medical information according to claim 3, further comprising:
a step of transmitting specific medicine data to the information management,institution from the research institution; and
a step of updating the database by the received medicine data in the information management institution.

7. The distribution method of the medical information according to claim 1, wherein a fixed global IP address is attached to the apparatus to obtain the medical information.

8. The distribution method of the medical information according to claim 2, wherein a fixed global IP address is attached to the apparatus to obtain the medical information.

9. The distribution method of the medical information according to claim 3, wherein a fixed global IP address is attached to the apparatus to obtain the medical information.

10. The distribution method of the medical information according to claim 1, further comprising:
a step of subjecting the medical information to an interpolation preventive measure and encryption.

11. The distribution method of the medical information according to claim 2, further comprising:
a step of subjecting the medical information to an interpolation preventive measure and encryption.

12. The distribution method of the medical information according to claim 3, further comprising:
a step of subjecting the medical information to an interpolation preventive measure and encryption.
